# DEMANDE DE BREVET EUROPEEN

(11) **EP 0 825 169 A1**
(43) Date de publication de la demande: **25.02.1998**
(21) Numéro de dépôt: 97401908.5
(22) Date de dépôt: 08.08.1997
(51) Int. Cl.: C07C 57/04, C08K 5/098, C07F 7/28

(54) **Composés métallo-organiques insaturés dérivés du titane et leur procédé de préparation**

(30) Priorité: 22.08.1996 FR 9610372
(71) Demandeur: ELF ATOCHEM S.A., 92800 Puteaux (FR)
(72) Inventeur: Riondel, Alain, 57600 Forbach (FR); Camail, Michel, 83170 Brignolles (FR); Margaillan, André, 83136 Gareoult (FR); Vernet, Jean-Louis, 83210 La Farlede (FR); Humbert, Marie, 13001 Marseille (FR)
(74) Mandataire: Rieux, Michel

(57) **Abrégé**

Ces composés sont représentés par la formule (I) : 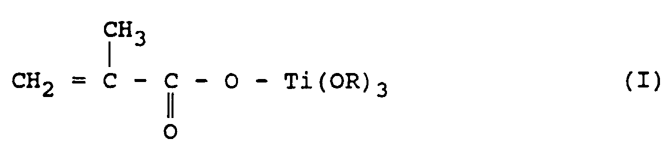 dans laquelle R représente un radical tertiobutyle, tertioamyle ou 2-éthylhexyle.

Pour les préparer, on fait réagir l'acide méthacrylique avec un tétraalcoxytitane Ti(OR)₄, où R = isopropyle ou tertiobutyle, pour obtenir un composé (I) correspondant, ou avec Ti(OR)(OtAm)₃, où R = isopropyle ou tertiobutyle et tAm = tertioamyle, pour obtenir un composé (I) avec R = tertioamyle, les composés (I) avec R = isopropyle ou tertiobutyle représentant par ailleurs des produits intermédiaires de synthèse que l'on peut faire réagir avec l'alcool tertioamylique ou avec le 2-éthylhexanol, pour obtenir un composé (I) dans lequel R représente respectivement tertioamyle ou 2-éthylhexyle.

## Description

La présente invention porte sur de nouveaux composés métallo-organiques insaturés dérivés du titane, ainsi que sur un procédé permettant de les préparer. Ces nouveaux composés sont applicables à la fabrication de nouveaux polymères qui peuvent notamment entrer dans la formulation de peintures, en particulier de peintures marines.

La présente invention a donc d'abord pour objet des composés métallo-organiques insaturés dérivés du titane, représentés par la formule (I) : 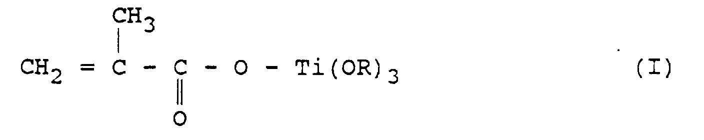 dans laquelle R représente un radical tertiobutyle, tertioamyle ou 2-éthylhexyle.

Pour préparer les composés (I), on fait réagir l'acide méthacrylique avec un tétraalcoxytitane Ti(OR)₄, où R = isopropyle ou tertiobutyle pour obtenir le composé (I) correspondant, ou avec Ti(OR)(OtAm)₃, où R = isopropyle ou tertiobutyle et tAm = tertioamyle, pour obtenir un composé (I) avec R = tertioamyle, les composés (I) avec R = isopropyle ou tertiobutyle représentant par ailleurs des produits intermédiaires de synthèse que l'on peut faire réagir avec l'alcool tertioamylique ou avec le 2-éthylhexanol, pour obtenir un composé (I) dans lequel R représente respectivement tertioamyle ou 2-éthylhexyle.

En particulier, on fait réagir l'acide méthacrylique avec Ti(OR)₄ ou Ti(OR)(OtAm)₃ avec R = isopropyle ou tertiobutyle et tAm = tertioamyle dans des conditions stoechiométriques, à une température de 40 - 80°C, sous une pression de 13,3 - 66,7 kPa (100 - 500 mmHg), l'acide méthacrylique étant ajouté à Ti(OR)₄ ou Ti(OR)(OtAm)₃, le cas échéant dans un solvant tel que le toluène, l'alcool ROH ou l'azéotrope ROH-solvant libéré par la réaction étant progressivement distillé, et lorsque l'on veut obtenir le composé de formule (I) avec R = tertioamyle ou 2-éthyl hexyle à partir des intermédiaires de synthèse, on poursuit la réaction en introduisant dans le milieu l'alcool tertio-amylique ou le 2-éthylhexanol à raison d'environ 3 moles pour 1 mole de composé intermédiaire (I) sous une pression de 13,3 - 66,7 kPa (100 - 500 mmHg) et à une température de 40 - 80°C, en distillant l'isopropanol ou le tertiobutanol libéré par la réaction.

On peut obtenir Ti(OR)(OtAm)₃ (R = isopropyle ou tertiobutyle et tAm = tertioamyle) en faisant réagir Ti(OR)₄ avec de l'alcool tertioamylique à raison de 3 moles de ce dernier pour 1 mole de Ti(OR)₄, sous une pression de 13,3-66,7 kPa (100 - 500 mmHg), à une température de 40- 80'C, en distillant l'isopropanol ou le tertiobutanol libéré par la réaction.

Les Exemples suivants illustrent la présente invention sans toutefois en limiter la portée.

### EXEMPLE 1 : Préparation du méthacrylate de tritertiobutoxytitane

Dans un ballon tricol de 100 ml, équipé d'une ampoule à addition isobare, d'un thermomètre et d'une colonne à distiller, on introduit 25,53 g de tétratertiobutoxytitane. La réaction est menée à bien sous 18 kPa (135 mmHg), le ballon réactionnel étant placé dans un bain d'huile à 55'C. Sous agitation magnétique, on ajoute goutte à goutte 6,13 g d'acide méthacrylique. Le tertiobutanol libéré par la réaction est progressivement distillé (point d'ébullition : 41°C/19 kPa (140 mmHg)). La réaction est suivie par une pesée du distillat libéré. En fin de réaction, on récupère dans le ballon le méthacrylate de tritertiobutoxytitane : c'est un liquide jaune pâle et peu visqueux.
Rendement : 97,5%.

### Analyse par RMN ¹H

δ_{H} (C₆D₆) en ppm :
1,4 [S, 27H, -CH₃]
1,7 [s, 3H, -CH₃]
5,2 et 6,3 [t, 2H, =CH₂].

### Analyse par RMN ¹³C

δ_{C} (C₆D₆) en ppm :
17,0 [-CH₃]
32,1 [-CH₃]
79,4 [Ti-O-C]
127,2 [=CH₂]
136,8 [=C]
185,2 [-CO₂-]

### Analyse IR (film)

2972, 2926 cm⁻¹: -CH₃(F)
1645 cm⁻¹ : C=C (f)
1588, 1525 cm⁻¹ : -COO- asym (m)
1426 cm⁻¹ : -COO- sym (m)
1005 cm⁻¹ : C-O (F, large)
638 cm⁻¹ : Ti-O (m)

### EXEMPLE 2 : Préparation du méthacrylate de tri(2-éthyl-hexoxy)titane

On conduit une première étape qui consiste à préparer le méthacrylate de triisopropoxytitane comme intermédiaire de synthèse, selon un mode opératoire décrit dans Tr. Inst. Khim., Akad. Nauk. SSR Ural. Filial. 13 39-47 (1968).

La deuxième étape de la synthèse est réalisée immédiatement à la suite de la première, sous une pression réduite de 15 kPa (110 mmHg) : par la seconde ampoule à addition, on introduit goutte à goutte, à chaud et sous agitation magnétique, 19,54 g de 2-éthylhexanol dans le ballon réactionnel contenant l'intermédiaire de synthèse. On distille rapidement l'isopropanol libéré par la réaction.

On récupère le produit attendu dans le ballon de la réaction ; il est liquide, visqueux, d'une couleur jaune soutenue.
Rendement : 98,5%.

### Analyse par RMN ¹H

δ_{H} (C₆D₆) en ppm :
0,9-1,1 [m, 18H, -CH₃]
1,1-1,9 [m, 25H, -CH₂- et CH]
2,1 [s, 3H, -CH₃]
4,6 [t, 6H, -CH₂-]
5,4 et 6,5 [t, 2H, =CH₂]

### Analyse par RMN ¹³C

δ_{C} (C₆D₆) en ppm :
11,4 [-CH₃]
14,4 [-CH₃]
18,9 [-CH₃]
23,5 [-CH₂-]
23,7 [-CH₂-]
29,8 [-CH₂-]
30,6 [-CH₂-]
43,2 [CH]
79,6 [O-CH₂]
124,8 [=CH₂]
139,2 [=C]
173,9 [-CO₂-]

### Analyse IR (film) :

2958, 2928, 2872 et 2859 cm⁻¹ : -CH₂- et -CH₃-(F)
1644 cm⁻¹ : C=C (f)
1559 cm⁻¹ : -COO- asym (m)
1423 cm⁻¹ : -COO- sym (m)
1088 cm⁻¹ : C-O (m, large)
673, 618 cm⁻¹ : Ti-O (m)

### EXEMPLE 3 : Préparation du méthacrylate de tritertioamyloxytitane

### Première étape

Dans un ballon tricol de 100 ml, équipé d'un chandelier avec deux ampoules à addition isobares, d'un thermomètre et d'une colonne à distiller, on introduit 14,21 g de tétraisopropoxytitane. On met alors le montage sous une pression réduite de 20 kPa (150 mmHg), et le ballon est placé dans un bain à 55'C. Sous agitation magnétique, on ajoute goutte à goutte 13,22 g d'alcool, et on distille l'isopropanol libéré par la réaction.

### Deuxième étape

La deuxième étape de la synthèse est réalisée immédiatement à la suite de la première, sous une pression réduite de 20 kPa (150 mmHg) : par la seconde ampoule à addition, on introduit goutte à goutte, à chaud et sous agitation magnétique 4,30 g d'acide méthacrylique. On distille rapidement l'isopropanol libéré par la réaction.

Le ballon contient, en fin de réaction, un liquide jaune clair, peu visqueux.
Rendement : 95,8%.

### Analyse par RMN ¹H

δ_{H} (C₆D₆) en ppm :
1,0 [t, 9H, -CH₃]
1,3 [s, 18H, -CH₃]
1,5 [m, 6H, -CH₂-]
1,8 [s, 3H, -CH₃]
5,2 et 6,3 [t, 2H, =CH₂]

### Analyse par RMN ¹³C

δ_{C} (C₆D₆) en ppm :
9,1 [-CH₃]
17,0 [-CH₃]
29,6 [-CH₃]
37,2 [-CH₂-]
80,0 [Ti-O-C]
127,1 [=CH₂]
136,8 [=C]
185,1 [-CO₂-]

### Analyse IR (film)

2970, 2926 cm⁻¹ : -CH₃ (F)
1644 cm⁻¹ : C=C (f)
1587, 1554, 1517 cm⁻¹ : -COO- asym (m)
1423 cm⁻¹ : -COO- sym (m)
1003 cm⁻¹ : C-O (F, large)
668, 623, 587 cm⁻¹ : Ti-O (m)

## Revendications

1. Composé métallo-organique insaturé dérivé du titane, représenté par la formule (I) : 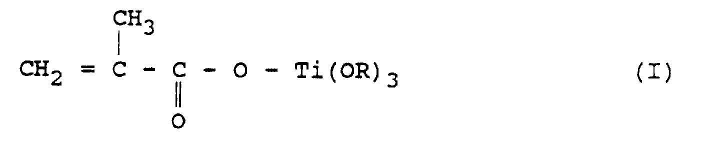 dans laquelle R représente un radical tertiobutyle, tertioamyle ou 2-éthylhexyle.

2. Procédé de fabrication d'un composé de formule (I) tel que défini à la revendication 1, caractérisé par le fait que l'on fait réagir l'acide méthacrylique avec un tétraalcoxytitane Ti(OR)₄, où R = isopropyle ou tertiobutyle, pour obtenir un composé (I) correspondant, ou avec Ti(OR)(OtAm)₃, où R = isopropyle ou tertiobutyle et tAm = tertioamyle, pour obtenir un composé (I) avec R = tertioamyle, les composés (I) avec R = isopropyle ou tertiobutyle représentant par ailleurs des produits intermédiaires de synthèse que l'on peut faire réagir avec l'alcool tertioamylique ou avec le 2-éthylhexanol, pour obtenir un composé (I) dans lequel R, représente respectivement tertioamyle ou 2-éthylhexyle.

3. Procédé selon la revendication 2, caractérisé par le fait que l'on fait réagir l'acide méthacrylique avec Ti(OR)₄ ou Ti(OR)(OtAm)₃ avec R = isopropyle ou tertiobutyle et tAm = tertioamyle dans des conditions stoechiométriques, à une température de 40 - 80°C, sous une pression de 13,3-66,7 kPa (100 - 500 mmHg), l'acide méthacrylique étant ajouté à Ti(OR)₄ ou Ti(OR)(OtAm)₃, le cas échéant dans un solvant tel que le toluène, l'alcool ROH ou l'azéotrope ROH-solvant libéré par la réaction étant progressivement distillé, et lorsque l'on veut obtenir le composé de formule (I) avec R = tertioamyle ou 2-éthyl hexyle à partir des intermédiaires de synthèse, on poursuit la réaction en introduisant dans le milieu l'alcool tertio-amylique ou le 2-éthylhexanol à raison d'environ 3 moles pour 1 mole de composé intermédiaire (I) sous une pression de 13,3 - 66,7 kPa (100 - 500 mmHg) et à une température de 40 - 80°C, en distillant l'isopropanol ou le tertiobutanol libéré par la réaction.

4. Procédé selon la revendication 3, caractérisé par le fait que l'on obtient Ti(OR)(OtAm)₃ (R = isopropyle ou tertiobutyle et tAm = tertioamyle) en faisant réagir Ti(OR)₄ avec de l'alcool tertioamylique à raison de 3 moles de ce dernier pour 1 mole de Ti(OR)₄, sous une pression de 13,3 - 66,7 kPa (100 - 500 mmHg), à une température de 40 - 80°C, en distillant l'isopropanol ou le tertiobutanol libéré par la réaction.
